# EUROPÄISCHE PATENTANMELDUNG

(11) **EP 1 921 070 A1**
(43) Veröffentlichungstag der Anmeldung: **14.05.2008**
(21) Anmeldenummer: 06123820.0
(22) Anmeldetag: 10.11.2006
(51) Int. Cl.: C07D 239/94, A61K 31/505, A61P 35/00

(54) **Bicyclische Heterocyclen, diese Verbindungen enthaltende Arzneimittel, deren Verwendung und Verfahren zu ihrer Herstelllung**

(71) Anmelder: Boehringer Ingelheim Pharma GmbH & Co. KG, 55216 Ingelheim (DE)
(72) Erfinder: Die Erfindernennung liegt noch nicht vor
(74) Vertreter: Hammann, Heinz

(57) **Zusammenfassung**

Die vorliegende Erfindung betrifft bicyclische Heterocyclen der allgemeinen Formel 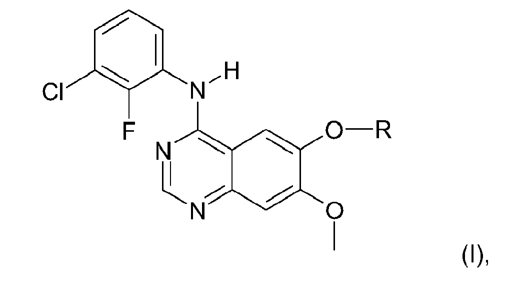 deren Tautomere, deren Stereoisomere, deren Gemische und deren Salze, insbesondere deren physiologisch verträgliche Salze mit anorganischen oder organischen Säuren, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege und deren Herstellung.

## Beschreibung

Gegenstand der vorliegenden Erfindung sind bicyclische Heterocyclen der allgemeinen Formel 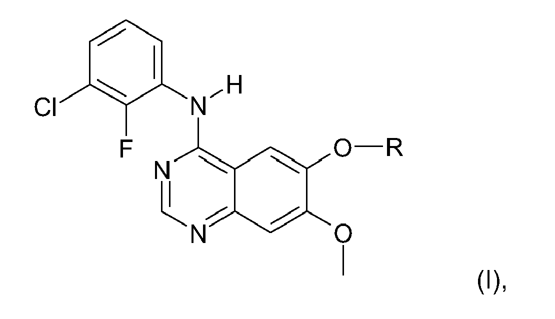 deren Tautomere, deren Stereoisomere, deren Gemische und deren Salze, insbesondere deren physiologisch verträgliche Salze mit anorganischen oder organischen Säuren, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege und deren Herstellung.

### In der obigen allgemeinen Formel (I) bedeutet

R ein Rest ausgewählt aus der Gruppe bestehend aus
cis-4-Amino-cyclohexyl, trans-4-Amino-cyclohexyl,
cis-4-Methylamino-cyclohexyl, trans-4-Methylamino-cyclohexyl,
cis-4-(Methoxycarbonylamino)-cyclohexyl, trans-4-(Methoxycarbonylamino)-cyclohexyl,
cis-4-(N-Methoxycarbonyl-N-methyl-amino)-cyclohexyl, trans-4-(N-Methoxycarbonyl-N-methyl-amino)-cyclohexyl,
cis-4-(Ethyloxycarbonylamino)-cyclohexyl, trans-4-(Ethyloxycarbonylamino)-cyclohexyl,
cis-4-(N-Ethyloxycarbonyl-N-methyl-amino)-cyclohexyl, trans-4-(N-Ethyloxycarbonyl-N-methyl-amino)-cyclohexyl,
cis-4-(Acetylamino)-cyclohexyl, trans-4-(Acetylamino)-cyclohexyl,
cis-4-(N-Acetyl-N-methyl-amino)-cyclohexyl, trans-4-(N-Acetyl-N-methyl-amino)-cyclohexyl,
cis-4-(Methoxyacetyl-amino)-cyclohexyl, trans-4-(Methoxyacetyl-amino)-cyclohexyl,
cis-4-(N-Methoxyacetyl-N-methyl-amino)-cyclohexyl, trans-4-(N-Methoxyacetyl-N-methyl-amino)-cyclohexyl,
cis-4-(Dimethylaminocarbonyl-amino)-cyclohexyl, trans-4-(Dimethylaminocarbonylamino)-cyclohexyl,
cis-4-(N-Dimethylaminocarbonyl-N-methyl-amino)-cyclohexyl, trans-4-(N-Dimethylaminocarbonyl-N-methyl-amino)-cyclohexyl,
cis-4-(Morpholinocarbonyl-amino)-cyclohexyl, trans-4-(Morpholinocarbonyl-amino)-cyclohexyl,
cis-4-(N-Morpholinocarbonyl-N-methyl-amino)-cyclohexyl, trans-4-(N-Morpholinocarbonyl-N-methyl-amino)-cyclohexyl,
cis-4-(Piperazin-1-ylcarbonyl-amino)-cyclohexyl, trans-4-(Piperazin-1-ylcarbonylamino)-cyclohexyl,
cis-4-(N-Piperazin-1-ylcarbonyl-N-methyl-amino)-cyclohexyl, trans-4-(N-Piperazin-1-ylcarbonyl-N-methyl-amino)-cyclohexyl,
cis-4-[(4-Methyl-piperazin-1-ylcarbonyl)-amino]-cyclohexyl, trans-4-[(4-Methylpiperazin-1-ylcarbonyl)-amino]-cyclohexyl,
cis-4-[N-(4-Methyl-piperazin-1-ylcarbonyl)-N-methyl-amino]-cyclohexyl, trans-4-[N-(4-Methyl-piperazin-1-ylcarbonyl)-N-methyl-amino]-cyclohexyl,
cis-4-(Methansulfonylamino)-cyclohexyl, trans-4-(Methansulfonylamino)-cyclohexyl,
cis-4-(N-Methansulfonyl-N-methyl-amino)-cyclohexyl, trans-4-(N-Methansulfonyl-N-methyl-amino)-cyclohexyl,
cis-4-Phthalimido-cyclohexyl und trans-4-Phthalimido-cyclohexyl,

gegebenenfalls in Form ihrer Tautomeren und ihrer Gemische, sowie gegebenenfalls ihrer pharmakologisch unbedenklichen Säureadditionssalze, Solvate und Hydrate.

Die Verbindungen der allgemeinen Formel (I) lassen sich beispielsweise nach folgenden Verfahren herstellen:
a) Umsetzung einer Verbindung der allgemeinen Formel 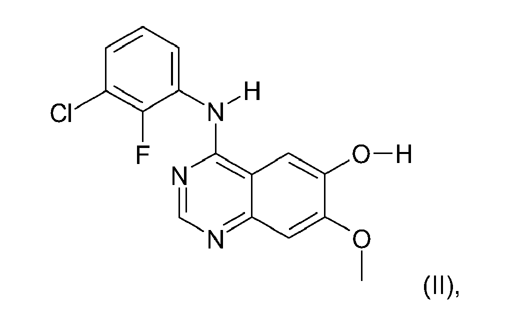 mit einer Verbindung der allgemeinen Formel

   Z¹ - R (III),

   in der
   R wie eingangs erwähnt definiert ist und Z¹ eine Austrittsgruppe wie ein Halogenatom, z.B. ein Chlor- oder Bromatom, eine Sulfonyloxygruppe wie eine Methansulfonyloxy- oder p-Toluolsulfonyloxygruppe oder eine Hydroxygruppe darstellt.
   Die Umsetzung erfolgt zweckmäßigerweise in einem Lösungsmittel wie Ethanol, Isopropanol, Acetonitril, Toluol, Tetrahydrofuran, Dioxan, Dimethylformamid, Dimethylsulfoxid oder N-Methylpyrrolidinon, gegebenfalls in Gegenwart einer Base wie Kaliumcarbonat oder N-Ethyl-diisopropylamin, bei Temperaturen im Bereich von 20°C bis 160°C, vorzugsweise bei Temperaturen im Bereich von 80°C bis 140°C.
   Mit einer Verbindung der allgemeinen Formel (III), in der Z¹ eine Hydroxygruppe darstellt, wird die Umsetzung in Gegenwart eines wasserentziehenden Mittels, vorzugsweise in Gegenwart eines Phosphins und eines Azodicarbonsäurederivates wie z.B. Triphenylphosphin/Azodicarbonsäurediethylester, zweckmäßigerweise in einem Lösungsmittel wie Methylenchlorid, Acetonitril, Tetrahydrofuran, Dioxan, Toluol oder Ethylenglycoldiethylether bei Temperaturen im Bereich von -50 bis 150°C, vorzugsweise jedoch bei Temperaturen im Bereich von -20 bis 80°C, durchgeführt.
b) Umsetzung einer Verbindung der allgemeinen Formel (IV) 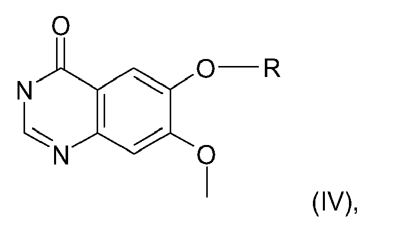 in der R wie eingangs erwähnt definiert ist, mit einem Halogenierungsmittel, beispielsweise einem Säurehalogenid wie Thionylchlorid, Thionylbromid, Phosphortrichlorid, Phosphorpentachlorid oder Phosphoroxychlorid zu einer Zwischenverbindung der allgemeinen Formel (V), 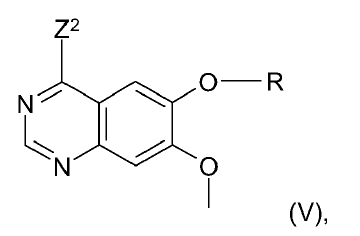 in der R wie eingangs erwähnt definiert ist und Z² ein Halogenatom wie ein Chlor- oder Bromatom darstellt,
   und anschließender Umsetzung mit 3-Chlor-2-fluor-anilin oder dessen Salzen. Die Umsetzung mit dem Halogenierungsmittel wird gegebenenfalls in einem Lösungsmittel wie Methylenchlorid, Chloroform, Acetonitril oder Toluol und gegebenfalls in Gegenwart einer Base wie N,N-Diethylanilin oder N-Ethyl-diisopropylamin bei Temperaturen im Bereich von 20°C bis 160°C, vorzugsweise von 40°C bis 120°C durchgeführt. Vorzugsweise wird die Reaktion jedoch mit Thionylchlorid und katalytischen Mengen an Dimethylformamid bei der Siedetemperatur des Reaktionsgemisches durchgeführt.
   Die Umsetzung der Verbindung der allgemeinen Formel (V) mit 3-Chlor-2-fluor-anilin oder dessen Salzen erfolgt zweckmäßigerweise in einem Lösungsmittel wie Ethanol, Isopropanol, Acetonitril, Dioxan oder Dimethylformamid, gegebenfalls in Gegenwart einer Base wie Kaliumcarbonat oder N-Ethyl-diisopropylamin, bei Temperaturen im Bereich von 20°C und 160°C, vorzugsweise von 60°C bis 120°C. Vorzugsweise wird die Reaktion jedoch in Isopropanol bei der Siedetemperatur des Reaktionsgemisches durchgeführt.
c) Zur Herstellung von Verbindungen der allgemeinen Formel (I), in der R ein Rest ausgewählt aus der Gruppe bestehend aus cis-4-(Methoxycarbonylamino)-cyclohexyl, trans-4-(Methoxycarbonylamino)-cyclohexyl, cis-4-(N-Methoxycarbonyl-N-methyl-amino)-cyclohexyl-, trans-4-(N-Methoxycarbonyl-N-methyl-amino)-cyclohexyl, cis-4-(Ethyloxycarbonylamino)-cyclohexyl, trans-4-(Ethyloxycarbonylamino)-cyclohexyl, cis-4-(N-Ethyloxycarbonyl-N-methyl-amino)-cyclohexyl, trans-4-(N-Ethyloxycarbonyl-N-methyl-amino)-cyclohexyl, cis-4-(Acetylamino)-cyclohexyl, trans-4-(Acetylamino)-cyclohexyl,cis-4-(N-Acetyl-N-methylamino)-cyclohexyl, trans-4-(N-Acetyl-N-methyl-amino)-cyclohexyl, cis-4-(Methoxyacetyl-amino)-cyclohexyl, trans-4-(Methoxyacetyl-amino)-cyclohexyl,cis-4-(N-Methoxyacetyl-N-methyl-amino)-cyclohexyl, trans-4-(N-Methoxyacetyl-N-methylamino)-cyclohexyl, cis-4-(Dimethylaminocarbonyl-amino)-cyclohexyl, trans-4-(Dimethylaminocarbonyl-amino)-cyclohexyl,cis-4-(N-Dimethylaminocarbonyl-N-methyl-amino)-cyclohexyl, trans-4-(N-Dimethylaminocarbonyl-N-methyl-amino)-cyclohexyl, cis-4-(Morpholinocarbonyl-amino)-cyclohexyl, trans-4-(Morpholinocarbonyl-amino)-cyclohexyl,cis-4-(N-Morpholinocarbonyl-N-methylamino)-cyclohexyl, trans-4-(N-Morpholinocarbonyl-N-methyl-amino)-cyclohexyl, cis-4-(Piperazin-1-ylcarbonyl-amino)-cyclohexyl, trans-4-(Piperazin-1-ylcarbonylamino)-cyclohexyl, cis-4-(N-Piperazin-1-ylcarbonyl-N-methyl-amino)-cyclohexyl, trans-4-(N-Piperazin-1-ylcarbonyl-N-methyl-amino)-cyclohexyl, cis-4-[(4-Methylpiperazin-1-ylcarbonyl)-amino]-cyclohexyl, trans-4-[(4-Methyl-piperazin-1-ylcarbonyl)-amino]-cyclohexyl, cis-4-[N-(4-Methyl-piperazin-1-ylcarbonyl)-N-methyl-amino]-cyclohexyl, trans-4-[N-(4-Methyl-piperazin-1-ylcarbonyl)-N-methyl-amino]-cyclohexyl, cis-4-(Methansulfonylamino)-cyclohexyl, trans-4-(Methansulfonylamino)-cyclohexyl, cis-4-(N-Methansulfonyl-N-methyl-amino)-cyclohexyl und trans-4-(N-Methansulfonyl-N-methyl-amino)-cyclohexyl bedeutet, Umsetzung einer Verbindung der allgemeinen Formel (V1) 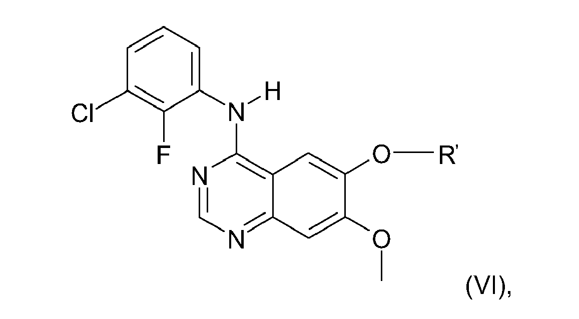 in der R' eine cis-4-Amino-cyclohex-1-yl-, trans-4-Amino-cyclohex-1-yl-, cis-4-(Methylamino)-cyclohex-1-yl- oder trans-4-(Methylamino)-cyclohex-1-yl-Gruppe darstellt,
   mit einem entsprechenden Acylierungsmittel wie Chlorameisensäure-methylester, Chlorameisensäure-ethylester, Pyrokohlensäure-dimethylester, Pyrokohlensäurediethylester, Acetanhydrid, Methoxyacetylchlorid, Dimethylcarbamoylchlorid, Morpholin-4-carbonylchlorid, 4-Methyl-piperazin-1-yl-carbonylchlorid, 4-(tert-Butyloxycarbonyl)-piperazin-1-yl-carbonylchlorid oder Methansulfonylchlorid. Die Umsetzung erfolgt zweckmäßigerweise in einem Lösungsmittel wie Methylenchlorid, Acetonitril, Toluol, Tetrahydrofuran, Dioxan, Dimethylformamid, Dimethylsulfoxid oder N-Methylpyrrolidinon, vorzugsweise in Tetrahydrofuran oder Dioxan, gegebenfalls in Gegenwart einer Base wie Kaliumcarbonat, Natronlauge oder N-Ethyl-diisopropylamin, bei Temperaturen im Bereich von -20°C bis 80°C, vorzugsweise von 0°C bis 40°C.
d) Zur Herstellung von Verbindungen der allgemeinen Formel (I), in der R eine cis-4-Phthalimido-cyclohex-1-yl- oder trans-4-Phthalimido-cyclohex-1-yl-Gruppe bedeutet, Umsetzung einer Verbindung der allgemeinen Formel (VII) 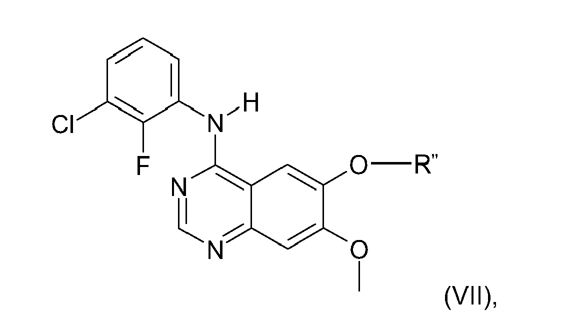 in der R" eine cis-4-Amino-cyclohexyl- oder trans-4-Amino-cyclohexyl-Gruppe darstellt,
   mit Phthalsäureanhydrid oder einem anderen reaktiven Derivat der Phthalsäure

Die Umsetzung erfolgt zweckmäßigerweise in einem Lösungsmittel wie Essigsäure, Acetonitril, Toluol, Tetrahydrofuran, Dioxan, Dimethylformamid, Dimethylsulfoxid oder N-Methylpyrrolidinon, vorzugsweise in Essigsäure, gegebenfalls in Gegenwart einer Base wie Kaliumcarbonat oder N-Ethyl-diisopropylamin, in einem Temperaturbereich von 60°C bis 160°C, vorzugsweise von 80°C bis 120°C.
Vorzugsweise wird die Reaktion jedoch in Essigsäure bei Temperaturen zwischen 80°C bis 120°C durchgeführt.

Bei den vorstehend beschriebenen Umsetzungen können gegebenenfalls vorhandene reaktive Gruppen wie Amino-, Alkylamino- oder Iminogruppen während der Umsetzung durch übliche Schutzgruppen geschützt werden, welche nach der Umsetzung wieder abgespalten werden.

Als Schutzreste für eine Amino-, Alkylamino- oder Iminogruppe kommen beispielsweise die Formyl-, Acetyl-, Trifluoracetyl-, Methoxycarbonyl, Ethoxycarbonyl-, tert.-Butoxycarbonyl-, Benzyloxycarbonyl-, Benzyl-, Methoxybenzyl- oder 2,4-Dimethoxybenzylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Die Abspaltung eines Methoxycarbonyl- oder Ethoxycarbonylrestes erfolgt vorzugsweise durch Behandlung mit Natronlauge, gegebenenfalls in Gegenwart eines Lösungsmittels wie Tetrahydrofuran oder Methanol bei Temperaturen zwischen 0 und 50°C.

Ferner können die erhaltenen Verbindungen der allgemeinen Formel I, wie bereits eingangs erwähnt wurde, in ihre Diastereomeren aufgetrennt werden. So können beispielsweise cis-/trans-Gemische durch Chromatographie oder Kristallisation in ihre cis- und trans-Isomere aufgetrennt werden.

Desweiteren können die erhaltenen Verbindungen der Formel (I) in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln (II) bis (VII) sind teilweise literaturbekannt (z.B. aus WO 03/82290 oder WO 03/082831) oder können nach an sich literaturbekannten Verfahren (siehe Beispiele I bis X), gegebenenfalls unter zusätzlicher Einführung von Schutzresten, erhalten werden.

Die erfindungsgemäßen Verbindungen der allgemeinen Formel (I) und ihre physiologisch verträglichen Salze weisen wertvolle pharmakologische Eigenschaften auf, insbesondere eine Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, daß die Signalübertragung an weiter abwärtsliegenden Komponenten blockiert wird.

Die biologischen Eigenschaften der neuen Verbindungen werden wie folgt geprüft:

Die Hemmung der humanen EGF-Rezeptorkinase wurde mit Hilfe der cytoplasmatischen Tyrosinkinase-Domäne (Methionin 664 bis Alanin 1186 basierend auf der in Nature 309 (1984), 418 publizierten Sequenz) bestimmt. Hierzu wurde das Protein in Sf9 Insektenzellen als GST-Fusionsprotein unter Verwendung des Baculovirus-Expressionssystems exprimiert.

Die Messung der Enzymaktivität wurde in Gegenwart oder Abwesenheit der Testverbindungen in seriellen Verdünnungen durchgeführt. Das Polymer pEY (4:1) von SIGMA wurde als Substrat verwendet. Biotinyliertes pEY (bio-pEY) wurde als Tracer-Substrat zugesetzt. Jede 100 µl Reaktionslösung enthielt 10 µl des Inhibitors in 50% DMSO, 20 µl der Substrat-Lösung (200 mM HEPES pH 7.4, 50 mM Magnesiumacetat, 2.5 mg/ml poly(EY), 5 µg/ml bio-pEY) und 20 µl Enzympräparation. Die Enzymreaktion wurde durch Zugabe von 50µl einer 100 µM ATP Lösung in 10 mM Magnesiumchlorid gestartet. Die Verdünnung der Enzympräparation wurde so eingestellt, daß der Phosphat-Einbau in das bio-pEY hinsichtlich Zeit und Enzymmenge linear war. Die Enzympräparation wurde in 20 mM HEPES pH 7.4, 1 mM EDTA, 130 mM Kochsalz, 0.05% Triton X-100, 1 mM DTT und 10% Glycerin verdünnt.

Die Enzymassays wurden bei Raumtemperatur über einen Zeitraum von 30 Minuten ausgeführt und durch Zugabe von 50 µl einer Stopplösung (250 mM EDTA in 20 mM HEPES pH 7.4) beendet. 100 µl wurden auf eine Streptavidin-beschichtete Mikrotiterplatte gebracht und 60 Minuten bei Raumtemperatur inkubiert. Danach wurde die Platte mit 200 µl einer Waschlösung (50 mM Tris, 0.05% Tween 20) gewaschen. Nach Zugabe von 100 µl eines HRPO-gelabelten anti-PY Antikörpers (PY20H Anti-PTyr:HRP von Transduction Laboratories, 250 ng/ml) wurde 60 Minuten inkubiert. Danach wurde die Mikrotiterplatte dreimal mit je 200 µl Waschlösung gewaschen. Die Proben wurden dann mit 100 µl einer TMB-Peroxidase-Lösung (A:B = 1:1, Kirkegaard Perry Laboratories) versetzt. Nach 10 Minuten wurde die Reaktion gestoppt. Die Extinktion wurde bei OD₄₅₀ₙₘ mit einem ELISA-Leser gemessen. Alle Datenpunkte wurden als Triplikate bestimmt.

Die Daten wurden mittels einer iterativen Rechnung unter Verwendung eines Analysenprogrammes für sigmoidale Kurven (Graph Pad Prism Version 3.0) mit variabler Hill-Steigung angepaßt. Alle freigegebenen Iterationsdaten wiesen einen Korrelationskoeffizienten von über 0.9 auf und die Ober- und Unterwerte der Kurven zeigten eine Spreizung von mindestens einem Faktor von 5. Aus den Kurven wurde die Wirkstoffkonzentration abgeleitet, die die Aktivität der EGF-Rezeptorkinase zu 50% hemmt (IC₅₀). Die erfindungsgemäßen Verbindungen weisen IC₅₀-Werte von unter 100 µM auf.

Die erfindungsgemäßen Verbindungen der allgemeinen Formel I hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuroepithelialen Ursprungs, Metastasierung sowie die abnorme Proliferation vaskulärer Endothelzellen (Neoangiogenese).

Die erfindungsgemäßen Verbindungen sind auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie akute Bronchitis, chronische Bronchitis, chronisch obstruktive Bronchitis (COPD), Asthma, Bronchiektasien, allergische oder nicht-allergische Rhinitis oder Sinusitis, zystische Fibrose, α1-Antitrypsin-Mangel, oder bei Husten, Lungenemphysem, Lungenfibrose und hyperreaktiven Atemwegen, sowie akute und chronische Erkrankungen der Nasenschleimhaut und Nasennebenhöhlen, wie akute und chronische Rhinitis, Sinusitis sowie Nasenpolypen.

Die Verbindungen sind auch geeignet für die Behandlung entzündlicher Erkrankungen des Magen-Darm-Traktes und der Gallengänge und -blase, die mit einer gestörten Aktivität der Tyrosinkinasen einhergehen, wie sie z.B. bei akuten oder chronisch entzündlichen Veränderungen zu finden sind, wie Cholezystitis, M. Crohn, Colitis ulcerosa, und Geschwüren oder Polyposis im Magen-Darm-Trakt oder wie sie bei Erkrankungen des Magen-Darm-Traktes, die mit einer vermehrten Sekretion einhergehen, vorkommen, wie M. Ménétrier, sezernierende Adenome und Proteinverlustsyndrome, desweiteren zur Behandlung von entzündlichen Erkrankungen der Gelenke, wie rheumatoider Arthritis, von entzündlichen Erkrankungen der Haut, der Augen, bei entzündlichen Pseudopolypen, bei Colitis cystica profunda oder bei Pneumatosis cystoides intestinales. Die Verbindungen kommen auch zur Behandlung von ZNS- und Rückenmarksverletzungen in Betracht.

Als bevorzugte Anwendungsgebiete seien entzündliche Erkrankungen der Atemwegsorgane oder des Darmes genannt, wie chronische Bronchitis (COPD), chronische Sinusitis, Asthma, M. Crohn, Colitis ulcerosa oder Polyposis des Darmes.

Besonders bevorzugte Anwendungsgebiete sind entzündliche Erkrankungen der Atemwege oder der Lunge wie chronische Bronchitis (COPD) oder Asthma, oder der Nasenschleimhaut und der Nebenhöhlenschleimhaut sowie Nasenpolypen.

Außerdem können die Verbindungen der allgemeinen Formel (I) und deren physiologisch verträglichen Salze zur Behandlung anderer Krankheiten verwendet werden, die durch aberrante Funktion von Tyrosinkinasen verursacht werden, wie z.B. epidermaler Hyperproliferation (Psoriasis), benigner Prostatahyperplasie (BPH), inflammatorischer Prozesse, Erkrankungen des Immunsystems, Hyperproliferation hämatopoetischer Zellen, der Behandlung von Nasenpolypen, etc..

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Für die Behandlung von Atemwegserkrankungen können diese Verbindungen allein oder in Kombination mit anderen Atemwegstherapeutika, wie z.B. sekretolytisch (z.B. Ambroxol, N-Acetylcystein), broncholytisch (z.B. Tiotropium oder Ipratropium oder Fenoterol, Salmeterol, Salbutamol) und/oder entzündungshemmend (z.B. Theophylline oder Glucocorticoide) wirksamen Substanzen angewendet werden. Für die Behandlung von Erkrankungen im Bereich des Magen-Darm-Traktes können diese Verbindungen ebenfalls alleine oder in Kombination mit Motilitäts- oder Sekretions-beeinflussenden Substanzen gegeben werden. Diese Kombinationen können entweder simultan oder sequentiell verabreicht werden.

Die Anwendung dieser Verbindungen entweder alleine oder in Kombination mit anderen Wirkstoffen kann intravenös, subkutan, intramuskulär, intraperitoneal, intranasal, durch Inhalation oder transdermal oder oral erfolgen, wobei zur Inhalation insbesondere Aerosolformulierungen geeignet sind.

Bei der pharmazeutischen Anwendung werden die erfindungsgemäßen Verbindungen in der Regel bei warmblütigen Wirbeltieren, insbesondere beim Menschen, in Dosierungen von 0.001-100 mg/kg Körpergewicht, vorzugsweise bei 0.1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragees, Kapseln, Pulver, Suspensionen, Lösungen, Sprays oder Zäpfchen eingearbeitet.

Die nachfolgenden Beispiele sollen die vorliegende Erfindung näher erläutern ohne diese zu beschränken:

### Herstellung der Ausgangsverbindungen:

### Beispiel I

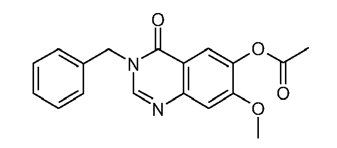

### 3-Benzyl-3,4-dihydro-4-oxo-6-acetyloxy-7-methoxy-chinazolin

169 g 3,4-Dihydro-4-oxo-6-acetyloxy-7-methoxy-chinazolin, 118.8 ml Benzylbromid und 138,2 g Kaliumcarbonat werden in 1600 ml Aceton für 8 Stunden auf 35-40°C erwärmt. Die Mischung wird 15 Stunden bei Raumtemperatur gerührt und anschließend mit 2000 ml Wasser versetzt. Die Suspension wird auf 0°C abgekühlt, der Niederschlag wird abgesaugt, mit 400 ml Wasser und 400 ml tert.-Butylmethylether gewaschen und bei 50°C getrocknet. Der Feststoff wird in 4000 ml Methylenchlorid gelöst, filtriert und eingeengt. Der Rückstand wird in tert.-Butylmethylether suspendiert, abgesaugt und bei 50°C getrocknet. Ausbeute: 203 g (86% der Theorie)
R_{f}-Wert: 0.80 (Kieselgel, Methylenchlorid/Ethanol = 9:1)
Massenspektrum (ESI⁺): m/z = 325 [M+H]⁺

### Beispiel II

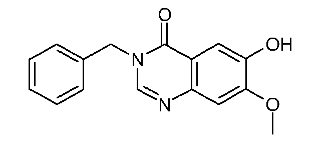

### 3-Benzyl-3,4-dihydro-4-oxo-6-hydroxy-7-methoxy-chinazolin

### Verfahren A:

168.5 g 6-Hydroxy-7-methoxy-benzo[d][1,3]oxazin-4-on werden in 1200 ml Toluol gelöst und 74.7 ml Benzylamin werden zugegeben. Die Mischung wird 15 Stunden unter Rückfluss erhitzt und danach auf Raumtemperatur abgekühlt. Der Niederschlag wird abfiltriert und mit tert.-Butylmethylether gewaschen.
Ausbeute 124 g (72% der Theorie)

### Verfahren B:

200 g 3-Benzyl-3,4-dihydro-4-oxo-6-acetyloxy-7-methoxy-chinazolin werden in 200 ml Wasser und 1000 ml Ethanol suspendiert. 300 ml 10N Natriumhydroxid Lösung werden bei Raumtemperatur zugegeben und die Mischung 1 Stunde auf 30°C erwärmt. Nach Zugabe von 172 ml Essigsäure und 2000 ml Wasser wird die Mischung 20 Stunden bei Raumtemperatur gerührt. Der Niederschlag wird abgesaugt, mit Wasser und Aceton gewaschen und bei 60°C getrocknet.
Ausbeute: 172,2 g (98% der Theorie)
R_{f}-Wert: 0.25 (Kieselgel, Methylenchlorid/Ethanol = 19:1)
Massenspektrum (ESI⁺): m/z = 283 [M+H]⁺

### Beispiel III

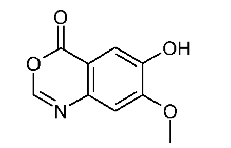

### 6-Hydroxy-7-methoxy-benzo[d][1,3]oxazin-4-on

1 g 2-Amino-5-hydroxy-4-methoxy-benzoesäure (hergestellt durch Umsetzung von 2-Nitro-4,5-dimethoxy-benzoesäure-methylester mit Kalilauge zu 2-Nitro-5-hydroxy-4-methoxy-benzoesäure-Kaliumsalz und anschließender katalytischer Hydrierung in Gegenwart von Palladium auf Aktivkohle) und 20 ml Orthoameisensäure-triethylester werden für 2.5 Stunden auf 100°C erhitzt. Nach Abkühlen auf Raumtemperatur wird der Niederschlag abgesaugt und mit Diethylether gewaschen.
Ausbeute: 0.97 g (93% der Theorie)
R_{f}-Wert: 0.86 (Kieselgel, Methylenchlorid/Methanol/Essigsäure = 90:10:1)
Massenspektrum (ESI⁺): m/z = 194 [M+H]⁺

### Beispiel IV

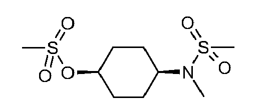

### cis-1-(Methansulfonyloxy)-4-(N-methansulfonyl-N-methyl-amino)-cyclohexan

Hergestellt durch Umsetzung von cis-1-Hydroxy-4-methylamino-cyclohexan mit Methansulfonsäurechlorid in Tetrahydrofuran in Gegenwart von Triethylamin.
Massenspektrum (ESI⁺): m/z = 286 [M+H]⁺

Analog Beispiel IV können erhalten werden:

| **Beispiel** | **Struktur** |
|---|---|
| IV(1) | 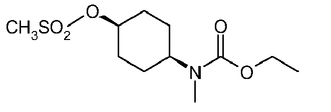 |
| IV(2) | 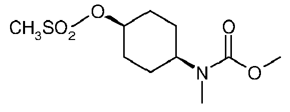 |
| IV(3) | 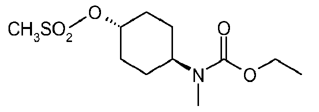 |
| IV(4) | 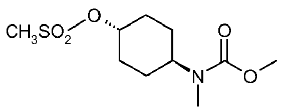 |
| IV(5) | 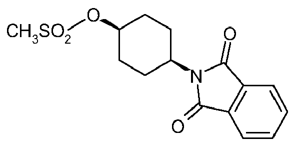 |
| IV(6) | 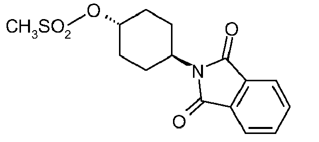 |
| IV(7) | 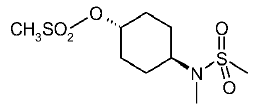 |
| IV(8) | 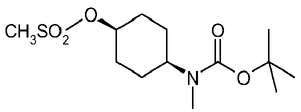 |
| | Massenspektrum (ESI⁺): m/z = 308 [M+H]⁺ |
| IV(9) | 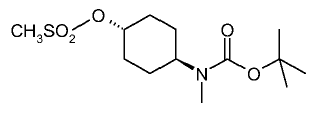 |
| IV(10) | 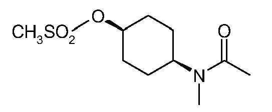 |
| IV(11) | 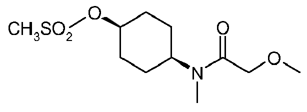 |
| IV(12) | 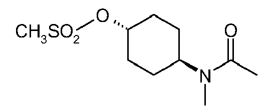 |
| IV(13) | 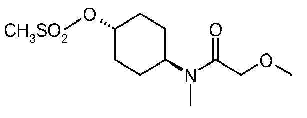 |

### Beispiel V

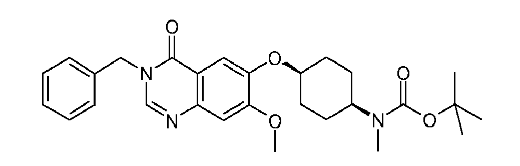

### 3-Benzyl-3,4-dihydro-4-oxo-6-{cis-4-[N-(tert-butyloxycarbonyl)-N-methyl-amino]-cyclohexyl-oxy}-7-methoxy-chinazolin

Hergestellt durch Umsetzung von 4 g 3-Benzyl-3,4-dihydro-4-oxo-6-hydroxy-7-methoxy-chinazolin und 8.77 g cis-1-Methansulfonyloxy-4-[N-(tert-butyloxycarbonyl)-N-methyl-amino]-cyclohexan in Gegenwart von 4.33 g Kaliumcarbonat in 32 ml N-Methyl-pyrrolidinon bei 100-120°C.
R_{f}-Wert: 0.78 (Kieselgel; Essigester/Methanol = 95:5) Massenspektrum (ESI⁺): m/z = 494 [M+H]⁺

### Analog Beispiel V können erhalten werden:

| **Beispiel** | **Struktur** |
|---|---|
| V(1) | 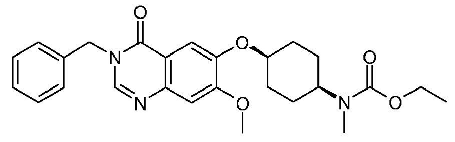 |
| V(2) | 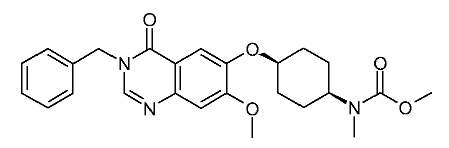 |
| V(3) | 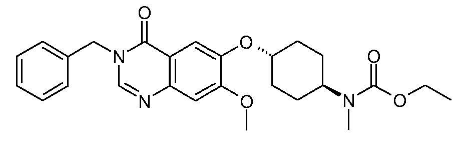 |
| V(4) | 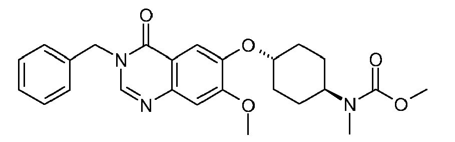 |
| V(5) | 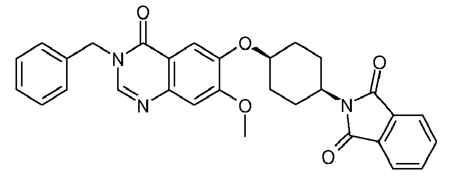 |
| V(6) | 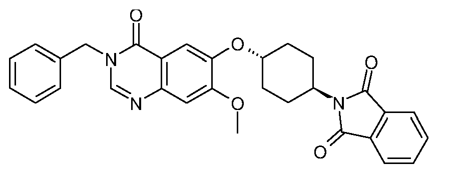 |
| V(7) | 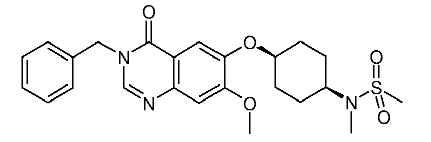 |
| V(8) | 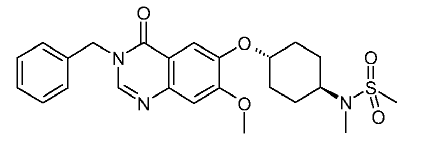 |
| V(9) | 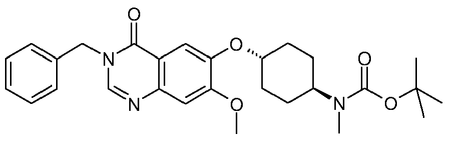 |
| V(10) | 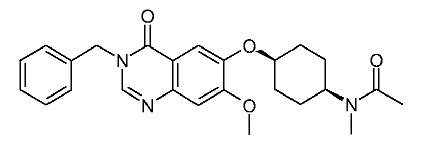 |
| V(11) | 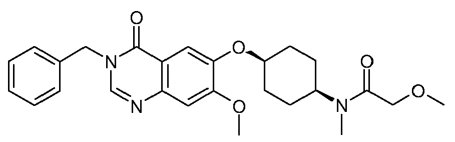 |
| V(12) | 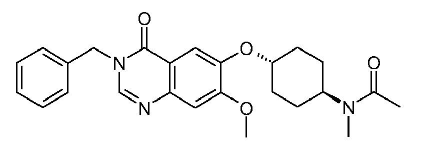 |
| V(13) | 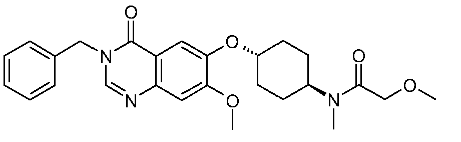 |

### Beispiel VI

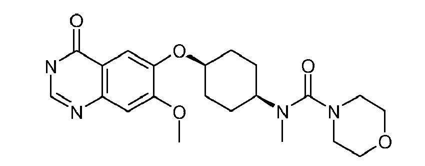

### 3,4-Dihydro-4-oxo-6-[cis-4-(N-morpholinocarbonyl-N-methyl-amino)-cyclohexyl-oxy]-7-methoxy-chinazolin

Eine Mischung aus 1.4 g 3-Benzyl-3,4-dihydro-4-oxo-6-[cis-4-(N-morpholinocarbonyl-N-methyl-amino)-cyclohexyl-oxy]-7-methoxy-chinazolin und 20 ml Eisessig werden in Gegenwart von 0.3 g Palladium auf Aktivkohle (10% Pd) bei 80°C und einem Wasserstoffdruck von 50 psi bis zur vollständigen Umsetzung hydriert. Der Katalysator wird abgesaugt, das Filtrat zur Trockene eingeengt und mit 15 ml Essigester versetzt. Der Niederschlag wird abgesaugt, mit 5 ml Essigester nachgewaschen und getrocknet. Ausbeute: 0.8 g (70% der Theorie)
R_{f}-Wert: 0.40 (Kieselgel, Methylenchlorid/Methanol = 9:1)
Massenspektrum (ESI⁺): m/z = 417 [M+H]⁺

### Analog Beispiel VI können erhalten werden:

| **Beispiel** | **Struktur** |
|---|---|
| VI(1) | 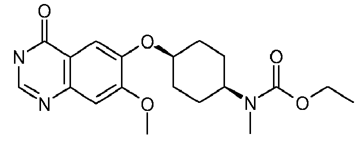 |
| VI(2) | 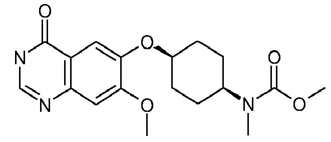 |
| VI(3) | 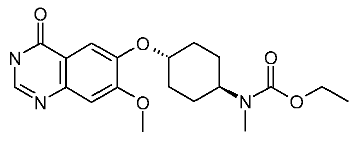 |
| VI(4) | 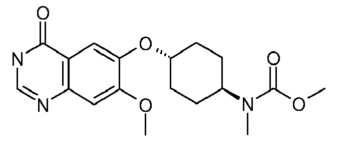 |
| VI(5) | 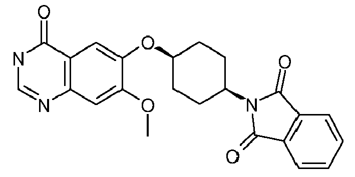 |
| VI(6) | 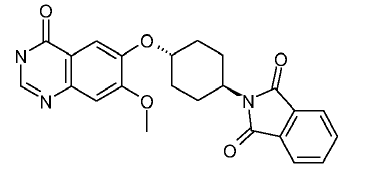 |
| VI(7) | 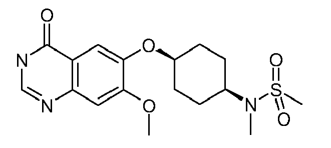 |
| VI(8) | 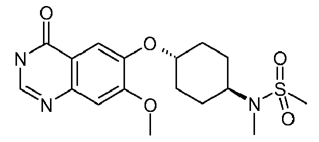 |
| VI(9) | 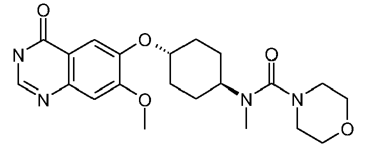 |
| VI(10) | 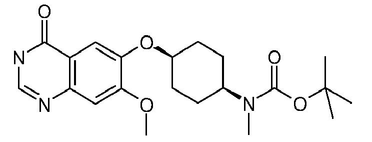 |
| VI(11) | 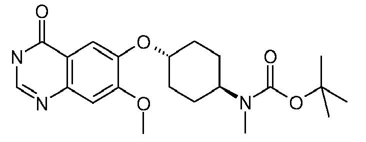 |
| VI(12) | 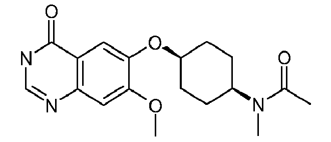 |
| VI(13) | 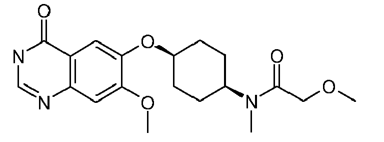 |
| VI(14) | 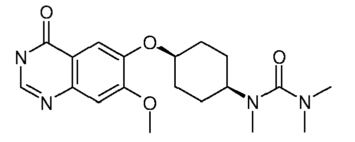 |
| VI(15) | 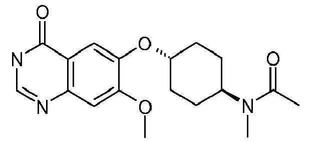 |
| VI(1l) | 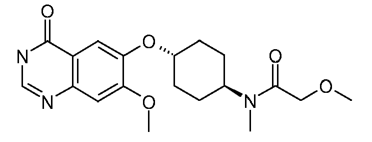 |
| VI(17) | 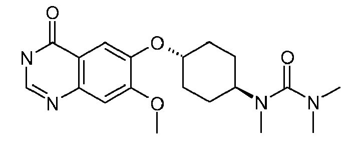 |

### Beispiel VII

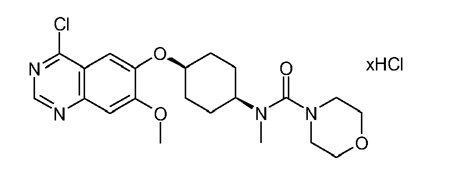

### 4-Chlor-6-[cis-4-(N-morpholinocarbonyl-N-methyl-amino)-cyclohexyl-oxy]-7-methoxychinazolin-hydrochlorid

800 mg 3,4-Dihydro-4-oxo-6-[cis-4-(N-morpholinocarbonyl-N-methyl-amino)-cyclohexyl-oxy]-7-methoxy-chinazolin, 7 ml Thionylchlorid und 0.1 ml Dimethylformamid werden für 3 Stunden unter Rückfluss erhitzt. Die flüchtigen Anteile des Reaktionsgemisches werden am Rotationsverdampfer abgezogen, der Rückstand mit Toluol versetzt und es wird erneut einrotiert.
Massenspektrum (ESI⁺): m/z = 435, 437 [M+H]⁺
Durch alkalische Aufarbeitung kann auch die freie Base erhalten werden.

### Analog Beispiel VII können erhalten werden:

| **Beispiel** | **Struktur** |
|---|---|
| VII(1) | 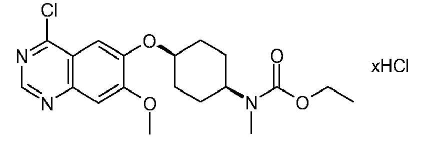 |
| VII(2) | 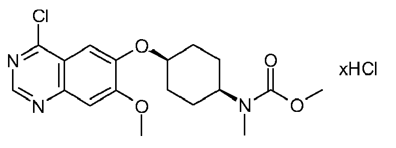 |
| VII(3) | 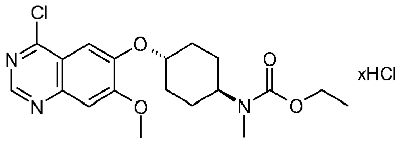 |
| VII(4) | 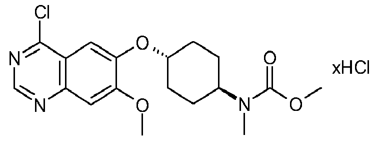 |
| VII(5) | 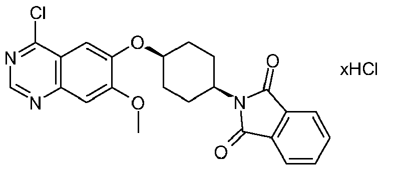 |
| VII(6) | 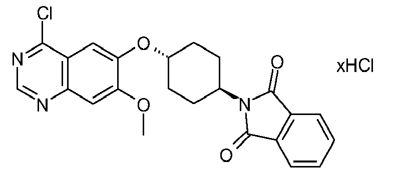 |
| VII(7) | 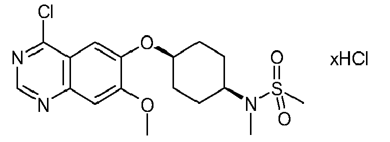 |
| VII(8) | 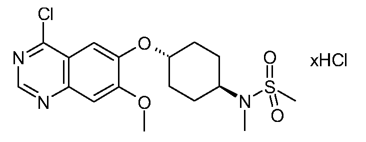 |
| VII(9) | 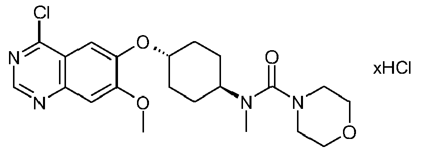 |
| VII(10) | 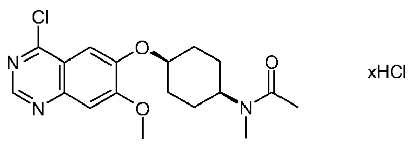 |
| VII(11) | 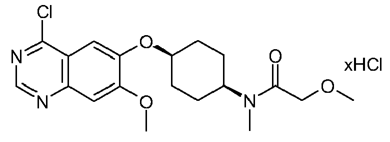 |
| VII(12) | 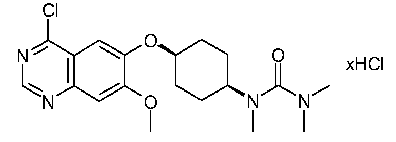 |
| VII(13) | 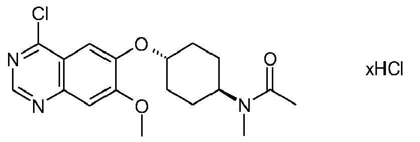 |
| VII(14) | 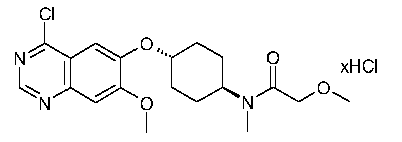 |
| VII(15) | 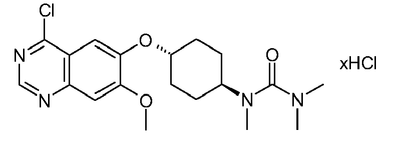 |

Durch alkalische Aufarbeitung können auch die freien Basen der vorstehend erwähnten Verbindungen erhalten werden.

### Beispiel VIII

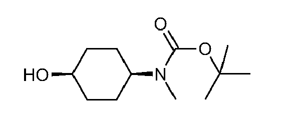

### cis-1-Hydroxy-4-(N-tert-butyloxycarbonyl-N-methyl-amino)-cyclohexan

Hergestellt durch Umsetzung von cis-1-Hydroxy-4-methylamino-cyclohexan mit Pyrokohlensäure-di-tert-butylester in Essigester bei Raumtemperatur.
Massenspektrum (ESI⁺): m/z = 230 [M+H]⁺

### Analog Beispiel VIII können erhalten werden:

| **Beispiel** | **Struktur** |
|---|---|
| VIII(1) | 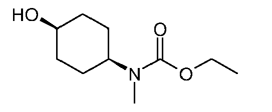 |
| VIII(2) | 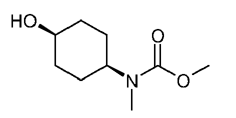 |
| VIII(3) | 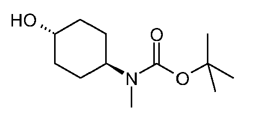 |
| VIII(4) | 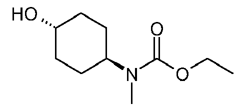 |
| VIII(5) | 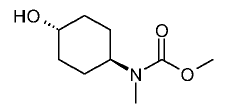 |
| VIII(6) | 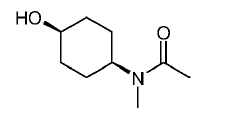 |
| VIII(7) | 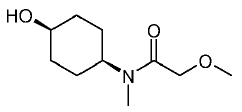 |
| VIII(8) | 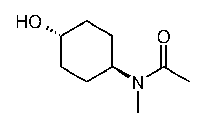 |
| VIII(9) | 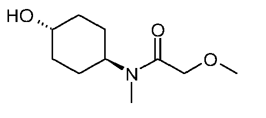 |

### Beispiel IX

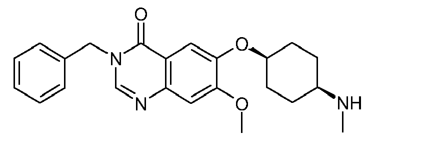

### 3-Benzyl-3,4-dihydro-4-oxo-6-(cis-4-methylamino-cyclohexan-1-yloxy)-7-methoxychinazolin-Hydrochlorid

Hergestellt durch Behandlung von 3-Benzyl-3,4-dihydro-4-oxo-6-{cis-4-[N-(tert-butyloxycarbonyl)-N-methyl-amino]-cyclohexyl-oxy}-7-methoxy-chinazolin mit isopropanolischer Salzsäure in Ethanol bei 40°C.
Massenspektrum (ESI⁺): m/z = 394 [M+H]⁺

### Analog Beispiel IX können erhalten werden:

| **Beispiel** | **Struktur** |
|---|---|
| IX(1) | 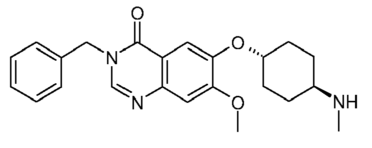 |
| IX(2) | 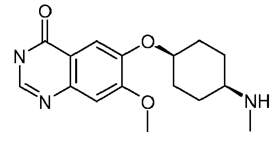 |
| IX(3) | 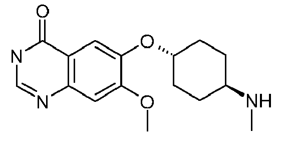 |

### Beispiel X

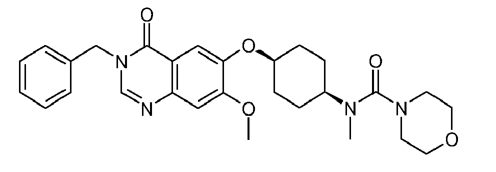

### 3-Benzyl-3,4-dihydro-4-oxo-6-[cis-4-(N-morpholinocarbonyl-N-methyl-amino)-cyclohexyl-oxy]-7-methoxy-chinazolin

Zu einem Gemisch aus 3 g 3-Benzyl-3,4-dihydro-4-oxo-6-(cis-4-methylaminocyclohexyl-oxy)-7-methoxy-chinazolin-Hydrochlorid, 2.67 ml N-Ethyl-diisopropylamin und 25 ml Acetonitril werden unter Kühlung im Eisbad 1.18 ml Morpholinocarbonylchlorid gelöst in 5 ml Acetonitril zugetropft. Nach Rühren bei Raumtemperatur über Nacht wird das Reaktionsgemisch zwischen 50 ml Wasser und 30 ml Essigester verteilt. Die Wasserphase wird mit 50 ml Essigester extrahiert und die vereinigten organischen Phasen mit 20 ml Wasser und gesättigter Kochsalzlösung gewaschen, getrocknet und eingeengt. Der Rückstand wird durch Chromatographie über eine Kieselgelsäule mit Methylenchlorid/Methanol (97:3 bis 95:5) gereinigt.
Ausbeute: 1.5 g (42% der Theorie)
R_{f}-Wert: 0.60 (Kieselgel; Methylenchlorid/Methanol = 9:1)
Massenspektrum (ESI⁺): m/z = 507 [M+H]⁺

### Analog Beispiel X können erhalten werden:

| **Beispiel** | **Struktur** |
|---|---|
| X(1) | 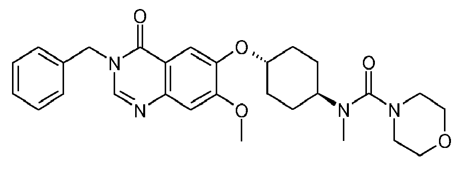 |
| X(2) | 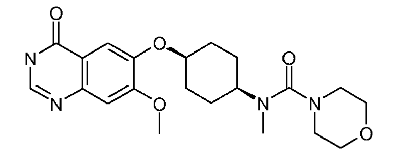 |
| X(3) | 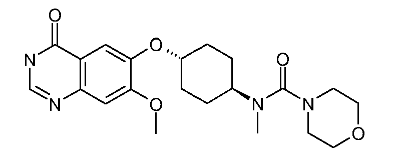 |
| X(4) | 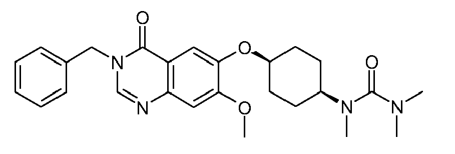 |
| X(5) | 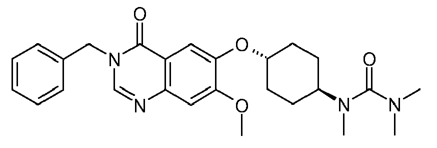 |
| X(6) | 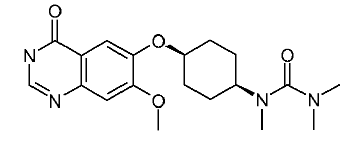 |
| X(7) | 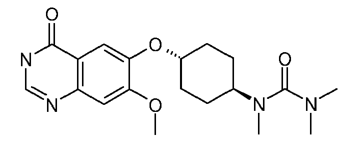 |
| X(8) | 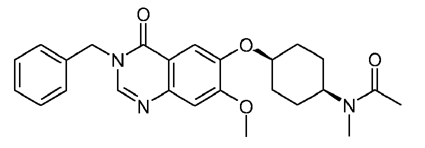 |
| X(9) | 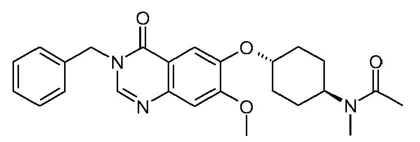 |
| X(10) | 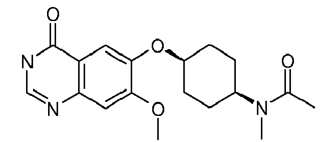 |
| X(11) | 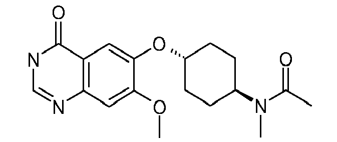 |
| | 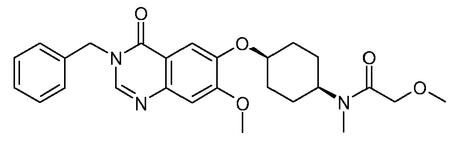 |
| X(13) | 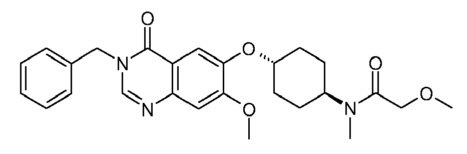 |
| X(14) | 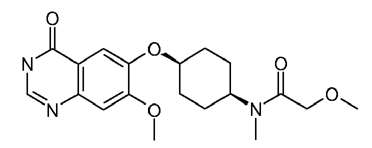 |
| X(15) | 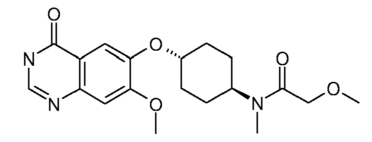 |

### Herstellung der Endverbindungen:

### Beispiel 1

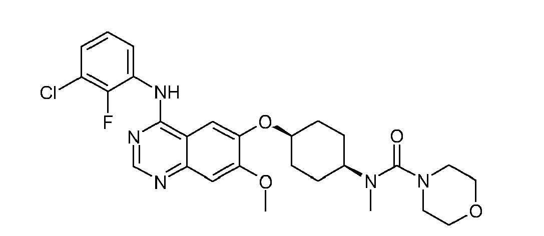

### 4-[(3-Chlor-2-fluor-phenyl)amino]-6-[cis-4-(N-morpholinocarbonyl-N-methyl-amino)-cyclohexyl-oxy]-7-methoxy-chinazolin

800 mg 3,4-Dihydro-4-oxo-6-[cis-4-(N-morpholinocarbonyl-N-methyl-amino)-cyclohexyl-oxy]-7-methoxy-chinazolin, 7 ml Thionylchlorid und 0.1 ml Dimethylformamid werden für 3 Stunden unter Rückfluss erhitzt. Die flüchtigen Anteile des Reaktionsgemisches werden am Rotationsverdampfer abgezogen, der Rückstand mit Toluol versetzt und es wird erneut einrotiert. Der Rückstand wird mit 30 ml Isopropanol und 643 mg 3-Chlor-2-fluor-anilin versetzt. Die Mischung wird 1.5 Stunden unter Rückfluss erhitzt. Danach wird zur Trockene eingeengt und der Rückstand zwischen 70 ml Essigester und 30 ml 10%iger wässriger Kaliumcarbonatlösung verteilt. Die organische Phase wird mit Wasser und Kochsalzlösung gewaschen, getrocknet und eingeengt. Der Rückstand wird durch Chromatographie über eine Kieselgelsäule mit Methylenchlorid/Methanol (95:5 bis 70:30) gereinigt.
Ausbeute: 580 mg (56% der Theorie)
R_{f}-Wert: 0.55 (Kieselgel; Methylenchlorid/Methanol = 7:1)
Massenspektrum (ESI⁺): m/z = 544, 546 [M+H]⁺

### Analog Beispiel 1 können erhalten werden:

| **Beispiel 1** | **Struktur** |
|---|---|
| (1) | 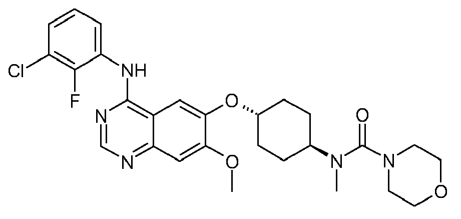 |
| (2) | 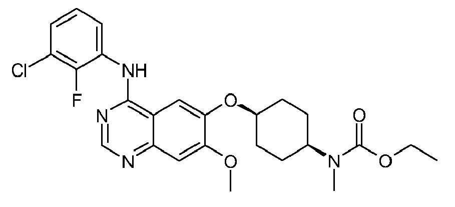 |
| (3) | 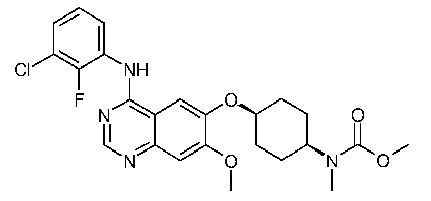 |
| (4) | 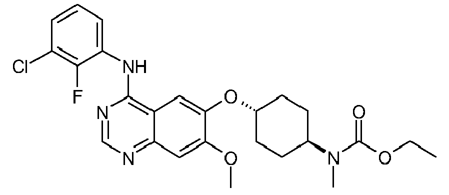 |
| (5) | 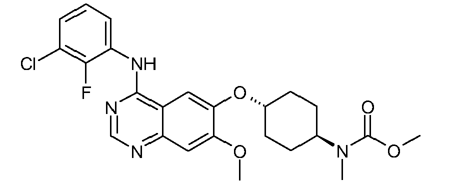 |
| (6) | 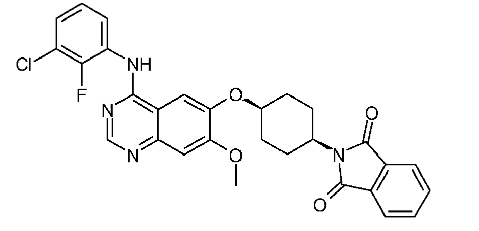 |
| (7) | 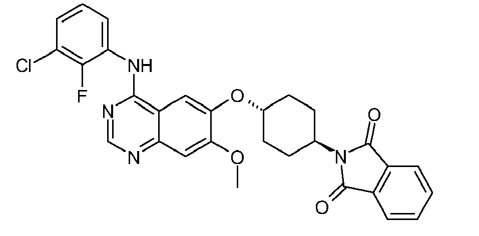 |
| (8) | 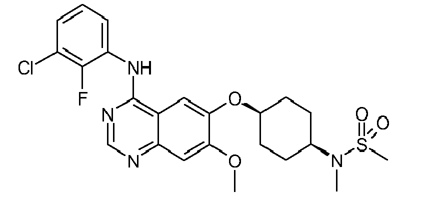 |
| (9) | 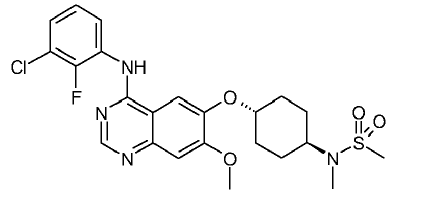 |
| (10) | 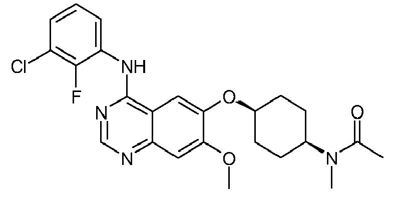 |
| (11) | 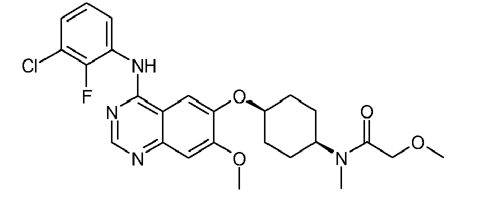 |
| (12) | 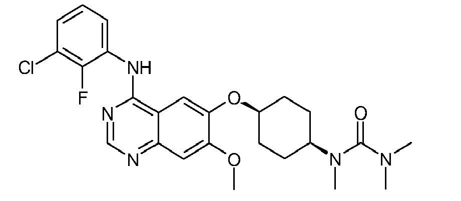 |
| (13) | 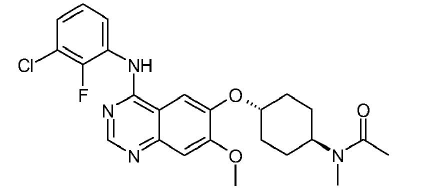 |
| (14) | 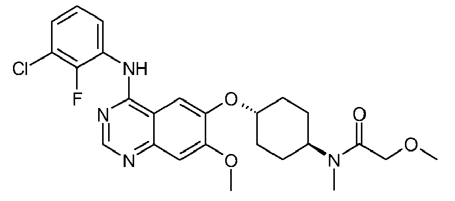 |
| (15) | 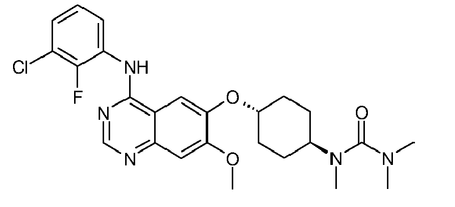 |

### Analog den vorstehend genannten Beispielen und anderen literaturbekannten Verfahren können auch folgende Verbindungen hergestellt werden:

| **Beispiel 1** | **Struktur** |
|---|---|
| (16) | 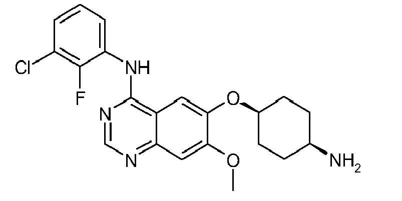 |
| (17) | 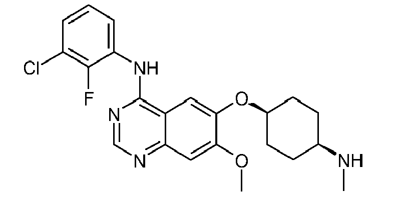 |
| (18) | 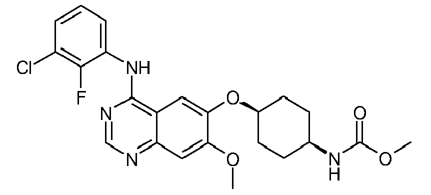 |
| (19) | 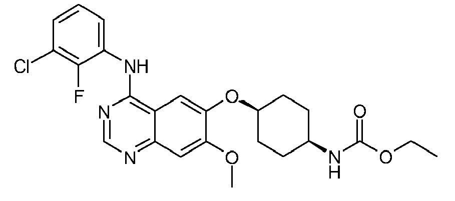 |
| (20) | 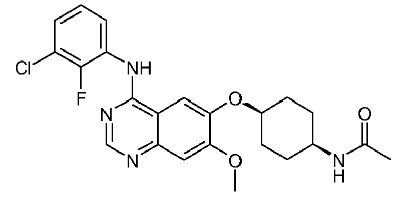 |
| (21) | 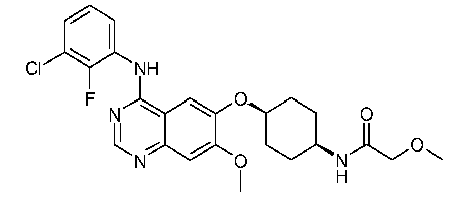 |
| (22) | 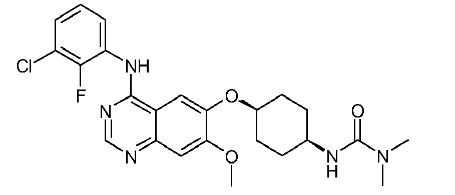 |
| (23) | 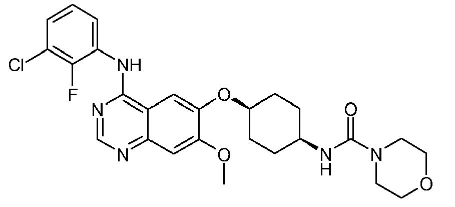 |
| (24) | 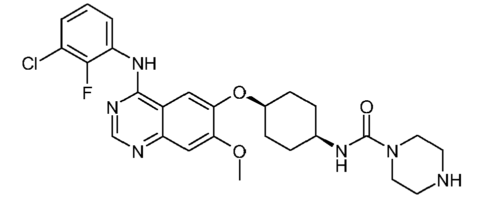 |
| (25) | 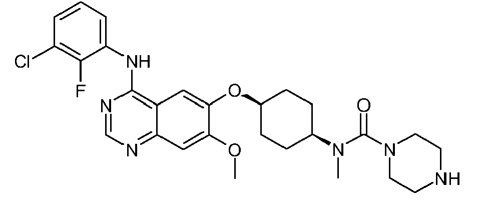 |
| (26) | 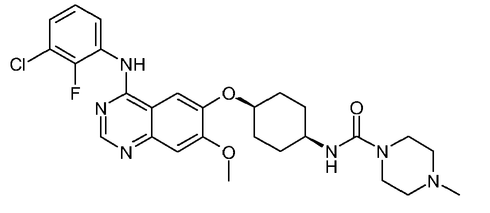 |
| (27) | 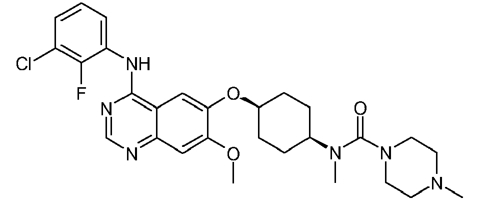 |
| (28) | 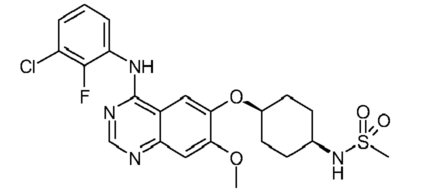 |
| (29) | 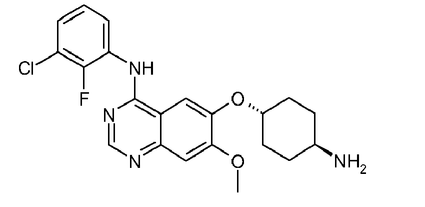 |
| (30) | 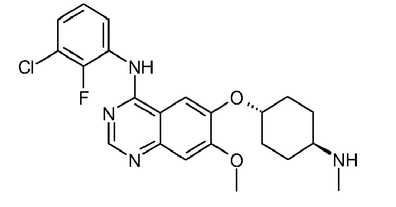 |
| (31) | 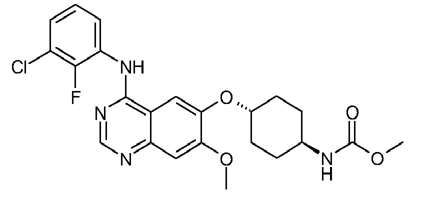 |
| (32) | 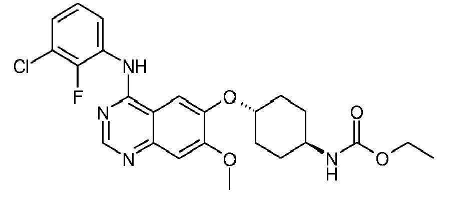 |
| (33) | 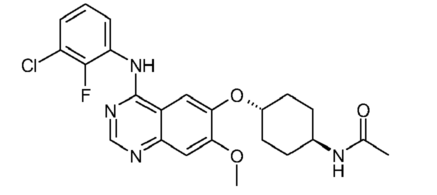 |
| (34) | 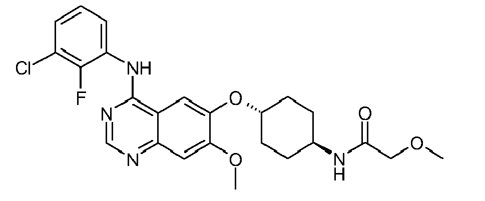 |
| (35) | 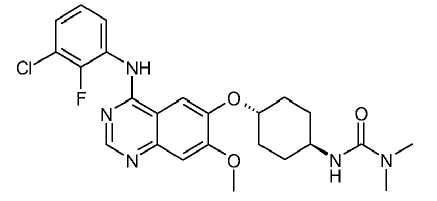 |
| (36) | 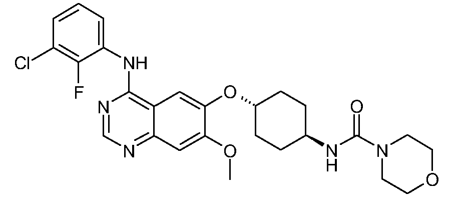 |
| (37) | 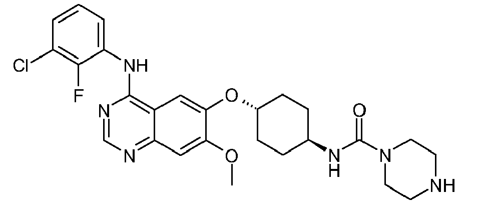 |
| (38) | 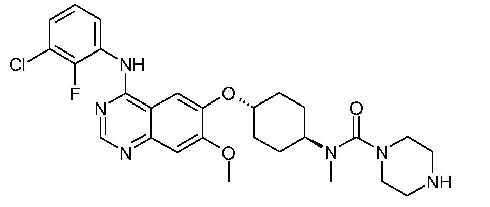 |
| (39) | 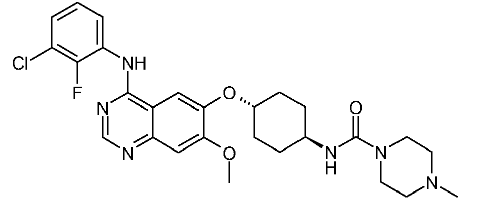 |
| (40) | 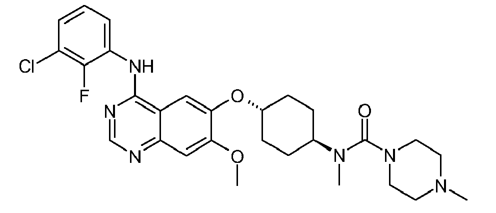 |
| (41) | 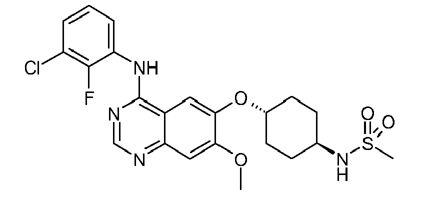 |

### Beispiel 2

### Dragees mit 75 mg Wirksubstanz

### Zusammensetzung:

| 1 | Dragéekern enthält: | |
|---|---|---|
| | Wirksubstanz | 75.0 mg |
| | Calciumphosphat | 93.0 mg |
| | Maisstärke | 35.5 mg |
| | Polyvinylpyrrolidon | 10.0 mg |
| | Hydroxypropylmethylcellulose | 15.0 mg |
| | Magnesiumstearat | 1.5 mg |
| | | 230.0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1.5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.

| | |
|---|---|
| Dragéegewicht: | 245 mg. |

### Beispiel 3

### Tabletten mit 100 mg Wirksubstanz

### Zusammensetzung:

| 1 | Tablette enthält: | |
|---|---|---|
| | Wirksubstanz | 100.0 mg |
| | Milchzucker | 80.0 mg |
| | Maisstärke | 34.0 mg |
| | Polyvinylpyrrolidon | 4.0 mg |
| | Magnesiumstearat | 2.0 mg |
| | | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 4

### Tabletten mit 150 mg Wirksubstanz

### Zusammensetzung:

| 1 | Tablette enthält: | |
|---|---|---|
| | Wirksubstanz | 150.0 mg |
| | Milchzucker pulv. | 89.0 mg |
| | Maisstärke | 40.0 mg |
| | Kolloide Kieselgelsäure | 10.0 mg |
| | Polyvinylpyrrolidon | 10.0 mg |
| | Magnesiumstearat | 1.0 mg |
| | | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 5

### Hartgelatine-Kapseln mit 150 mg Wirksubstanz

### Zusammensetzung:

| 1 | Kapsel enthält: | |
|---|---|---|
| | Wirkstoff | 150.0 mg |
| | Maisstärke getr. ca. | 180.0 mg |
| | Milchzucker pulv. ca. | 87.0 mg |
| | Magnesiumstearat | 3.0 mg |
| | ca. | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.
Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel 6

### Suppositorien mit 150 mg Wirksubstanz

### Zusammensetzung:

| 1 | Zäpfchen enthält: | |
|---|---|---|
| | Wirkstoff | 150.0 mg |
| | Polyäthylenglykol 1500 | 550.0 mg |
| | Polyäthylenglykol 6000 | 460.0 mg |
| | Polyoxyäthylensorbitanmonostearat | 840.0 mg |
| | | 2000.0 mg |

### Herstellung:

Nach dem Aufschmelzen der Suppositorienmasse wird der Wirkstoff darin homogen verteilt und die Schmelze in vorgekühlte Formen gegossen.

### Beispiel 7

### Suspension mit 50 mg Wirksubstanz

### Zusammensetzung:

| 100 ml Suspension enthalten: | |
|---|---|
| Wirkstoff | 1.00 g |
| Carboxymethylcellulose-Na-Salz | 0.10 g |
| p-Hydroxybenzoesäuremethylester | 0.05 g |
| p-Hydroxybenzoesäurepropylester | 0.01 g |
| Rohrzucker | 10.00 g |
| Glycerin | 5.00 g |
| Sorbitlösung 70%ig | 20.00 g |
| Aroma | 0.30 g |
| Wasser dest.ad 100.00 ml | |

### Herstellung:

Destilliertes Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 8

### Ampullen mit 10 mg Wirksubstanz

### Zusammensetzung:

| | |
|---|---|
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### Beispiel 9

### Ampullen mit 50 mg Wirksubstanz

### Zusammensetzung:

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

### Beispiel 10

### Kapseln zur Pulverinhalation mit 5 mg Wirksubstanz

| 1 | Kapsel enthält: | |
|---|---|---|
| | Wirksubstanz | 5.0 mg |
| | Lactose für Inhalationszwecke | 15.0 mg |
| | | 20.0 mg |

### Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.

| | |
|---|---|
| Kapselgewicht: | 70.0 mg |
| Kapselgröße: | 3 |

### Beispiel 11

### Inhalationslösung für Handvernebler mit 2.5 mg Wirksubstanz

| 1 | Hub enthält: | |
|---|---|---|
| | Wirksubstanz | 2.500 mg |
| | Benzalkoniumchlorid | 0.001 mg |
| | 1N-Salzsäure q.s. | |
| | Ethanol/Wasser (50/50) ad | 15.000 mg |

### Herstellung:

Die Wirksubstanz und Benzalkoniumchlorid werden in Ethanol/Wasser (50/50) gelöst. Der pH-Wert der Lösung wird mit 1N-Salzsäure eingestellt. Die eingestellte Lösung wird filtriert und in für den Handvernebler geeignete Behälter (Kartuschen) abgefüllt.

Füllmasse des Behälters: 4.5 g

## Patentansprüche

**1.** Bicyclische Heterocyclen der allgemeinen Formel (I) 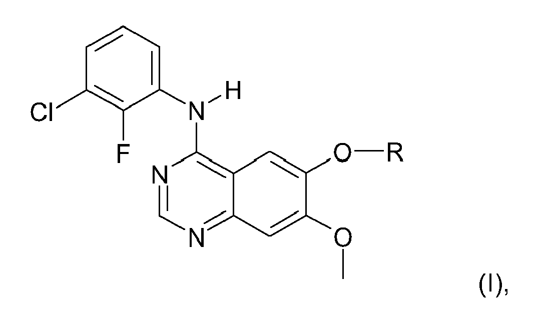 worin
R ein Rest ausgewählt aus der Gruppe bestehend aus
cis-4-Amino-cyclohexyl, trans-4-Amino-cyclohexyl,
cis-4-Methylamino-cyclohexyl, trans-4-Methylamino-cyclohexyl,
cis-4-(Methoxycarbonylamino)-cyclohexyl, trans-4-(Methoxycarbonylamino)-cyclohexyl,
cis-4-(N-Methoxycarbonyl-N-methyl-amino)-cyclohexyl-, trans-4-(N-Methoxycarbonyl-N-methyl-amino)-cyclohexyl,
cis-4-(Ethyloxycarbonylamino)-cyclohexyl, trans-4-(Ethyloxycarbonylamino)-cyclohexyl,
cis-4-(N-Ethyloxycarbonyl-N-methyl-amino)-cyclohexyl, trans-4-(N-Ethyloxycarbonyl-N-methyl-amino)-cyclohexyl,
cis-4-(Acetylamino)-cyclohexyl, trans-4-(Acetylamino)-cyclohexyl,
cis-4-(N-Acetyl-N-methyl-amino)-cyclohexyl, trans-4-(N-Acetyl-N-methyl-amino)-cyclohexyl,
cis-4-(Methoxyacetyl-amino)-cyclohexyl, trans-4-(Methoxyacetyl-amino)-cyclohexyl,
cis-4-(N-Methoxyacetyl-N-methyl-amino)-cyclohexyl, trans-4-(N-Methoxyacetyl-N-methyl-amino)-cyclohexyl,
cis-4-(Dimethylaminocarbonyl-amino)-cyclohexyl, trans-4-(Dimethylaminocarbonylamino)-cyclohexyl,
cis-4-(N-Dimethylaminocarbonyl-N-methyl-amino)-cyclohexyl, trans-4-(N-Dimethylaminocarbonyl-N-methyl-amino)-cyclohexyl,
cis-4-(Morpholinocarbonyl-amino)-cyclohexyl, trans-4-(Morpholinocarbonyl-amino)-cyclohexyl,
cis-4-(N-Morpholinocarbonyl-N-methyl-amino)-cyclohexyl, trans-4-(N-Morpholinocarbonyl-N-methyl-amino)-cyclohexyl,
cis-4-(Piperazin-1-ylcarbonyl-amino)-cyclohexyl, trans-4-(Piperazin-1-ylcarbonylamino)-cyclohexyl,
cis-4-(N-Piperazin-1-ylcarbonyl-N-methyl-amino)-cyclohexyl, trans-4-(N-Piperazin-1-ylcarbonyl-N-methyl-amino)-cyclohexyl,
cis-4-[(4-Methyl-piperazin-1-ylcarbonyl)-amino]-cyclohexyl, trans-4-[(4-Methylpiperazin-1-ylcarbonyl)-amino]-cyclohexyl,
cis-4-[N-(4-Methyl-piperazin-1-ylcarbonyl)-N-methyl-amino]-cyclohexyl, trans-4-[N-(4-Methyl-piperazin-1-ylcarbonyl)-N-methyl-amino]-cyclohexyl,
cis-4-(Methansulfonylamino)-cyclohexyl, trans-4-(Methansulfonylamino)-cyclohexyl,
cis-4-(N-Methansulfonyl-N-methyl-amino)-cyclohexyl, trans-4-(N-Methansulfonyl-N-methyl-amino)-cyclohexyl, cis-4-Phthalimido-cyclohexyl und trans-4-Phthalimidocyclohexyl,
gegebenenfalls in Form ihrer Tautomeren und ihrer Gemische, sowie gegebenenfalls ihrer pharmakologisch unbedenklichen Säureadditionssalze, Solvate und Hydrate, bedeutet.

**2.** Physiologisch verträgliche Salze der Verbindungen gemäß Anspruch 1 mit anorganischen oder organischen Säuren.

**3.** Arzneimittel, enthaltend eine Verbindung gemäß Anspruch 1 oder ein physiologisch verträgliches Salz gemäß Anspruch 2 neben gegebenenfalls einem oder mehreren inerten Trägerstoffen und/oder Verdünnungsmitteln.

**4.** Verwendung einer Verbindung gemäß mindestens einem der Ansprüche 1 bis 2 zur Herstellung eines Arzneimittels, das zur Behandlung von benignen oder malignen Tumoren, zur Vorbeugung und Behandlung von Erkrankungen der Atemwege und der Lunge sowie zur Behandlung von Erkrankungen des Magen-Darm-Traktes und der Gallengänge und -blase geeignet ist.

**5.** Verfahren zur Herstellung der Verbindungen der allgemeinen Formel I gemäß Anspruch 1, **dadurch gekennzeichnet, dass**
a) eine Verbindung der allgemeinen Formel (II) 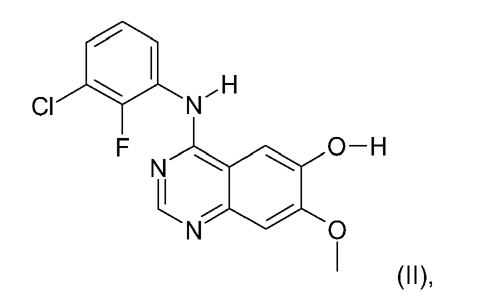 mit einer Verbindung der allgemeinen Formel (III)
Z¹ - R (III),
worin,
R wie im Anspruch 1 definiert ist, und
Z¹ eine Austrittsgruppe oder Hydroxygruppe darstellt, umgesetzt wird, oder
b) eine Verbindung der allgemeinen Formel (IV) 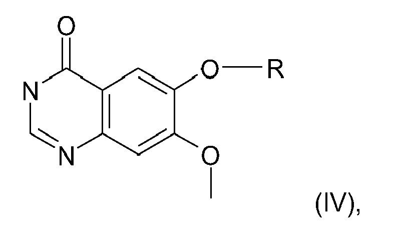 in der R wie im Anspruch 1 definiert ist,
mit einem Halogenierungsmittel zu einer Zwischenverbindung der allgemeinen Formel (V), 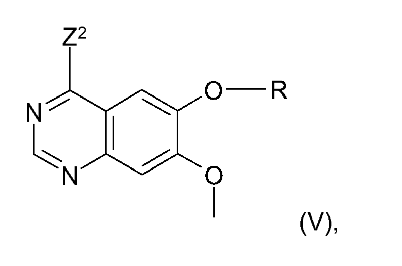 in der R wie im Anspruch 1 definiert ist, und Z² ein Halogenatom darstellt, umgesetzt wird, und anschließend mit 3-Chlor-2-fluor-anilin umgesetzt wird.

**7.** Verfahren zur Herstellung der Verbindungen der allgemeinen Formel (I) gemäß Anspruch 1, **dadurch gekennzeichnet, dass**
R ein Rest ausgewählt aus der Gruppe bestehend aus cis-4-(Methoxycarbonylamino)-cyclohexyl, trans-4-(Methoxycarbonylamino)-cyclohexyl, cis-4-(N-Methoxycarbonyl-N-methyl-amino)-cyclohexyl-, trans-4-(N-Methoxycarbonyl-N-methyl-amino)-cyclohexyl, cis-4-(Ethyloxycarbonylamino)-cyclohexyl, trans-4-(Ethyloxycarbonylamino)-cyclohexyl, cis-4-(N-Ethyloxycarbonyl-N-methyl-amino)-cyclohexyl, trans-4-(N-Ethyloxycarbonyl-N-methyl-amino)-cyclohexyl, cis-4-(Acetylamino)-cyclohexyl, trans-4-(Acetylamino)-cyclohexyl,cis-4-(N-Acetyl-N-methylamino)-cyclohexyl, trans-4-(N-Acetyl-N-methyl-amino)-cyclohexyl, cis-4-(Methoxyacetyl-amino)-cyclohexyl, trans-4-(Methoxyacetyl-amino)-cyclohexyl,cis-4-(N-Methoxyacetyl-N-methyl-amino)-cyclohexyl, trans-4-(N-Methoxyacetyl-N-methylamino)-cyclohexyl, cis-4-(Dimethylaminocarbonyl-amino)-cyclohexyl, trans-4-(Dimethylaminocarbonyl-amino)-cyclohexyl,cis-4-(N-Dimethylaminocarbonyl-N-methyl-amino)-cyclohexyl, trans-4-(N-Dimethylaminocarbonyl-N-methyl-amino)-cyclohexyl, cis-4-(Morpholinocarbonyl-amino)-cyclohexyl, trans-4-(Morpholinocarbonyl-amino)-cyclohexyl,cis-4-(N-Morpholinocarbonyl-N-methylamino)-cyclohexyl, trans-4-(N-Morpholinocarbonyl-N-methyl-amino)-cyclohexyl, cis-4-(Piperazin-1-ylcarbonyl-amino)-cyclohexyl, trans-4-(Piperazin-1-ylcarbonylamino)-cyclohexyl,
cis-4-(N-Piperazin-1-ylcarbonyl-N-methyl-amino)-cyclohexyl, trans-4-(N-Piperazin-1-ylcarbonyl-N-methyl-amino)-cyclohexyl, cis-4-[(4-Methyl-piperazin-1-ylcarbonyl)-amino]-cyclohexyl, trans-4-[(4-Methyl-piperazin-1-ylcarbonyl)-amino]-cyclohexyl, cis-4-[N-(4-Methyl-piperazin-1-ylcarbonyl)-N-methyl-amino]-cyclohexyl, trans-4-[N-(4-Methyl-piperazin-1-ylcarbonyl)-N-methyl-amino]-cyclohexyl, cis-4-(Methansulfonylamino)-cyclohexyl, trans-4-(Methansulfonylamino)-cyclohexyl, cis-4-(N-Methansulfonyl-N-methyl-amino)-cyclohexyl und trans-4-(N-Methansulfonyl-N-methyl-amino)-cyclohexyl,
bedeutet,
eine Verbindung der allgemeinen Formel (VI) 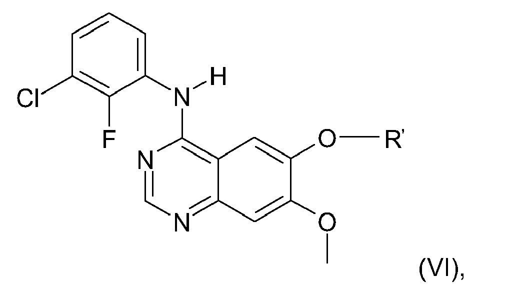 worin
R' ein Rest ausgewählt aus der Gruppe bestehend aus cis-4-Amino-cyclohex-1-yl, trans-4-Amino-cyclohex-1-yl, cis-4-(Methylamino)-cyclohex-1-yl und trans-4-(Methylamino)-cyclohex-1-yl, darstellt, mit einem entsprechenden Acylierungsmittel umgesetzt wird.

**8.** Verfahren zur Herstellung der Verbindungen der allgemeinen Formel (I) gemäß Anspruch 1 , **dadurch gekennzeichnet, dass**
R eine cis-4-Phthalimido-cyclohex-1-yl- oder trans-4-Phthalimido-cyclohex-1-yl-Gruppe bedeutet, und
eine Verbindung der allgemeinen Formel (VII) 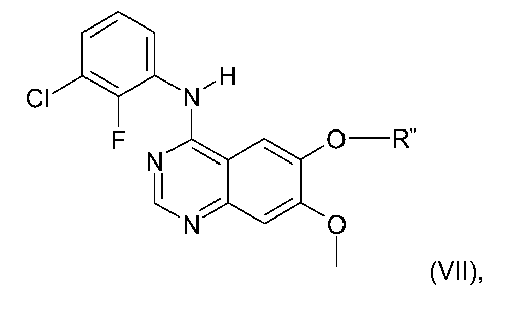 worin,
R" eine cis-4-Amino-cyclohex-1-yl- oder trans-4-Amino-cyclohex-1-yl-Gruppe darstellt,
mit Phthalsäureanhydrid oder einem anderen reaktiven Derivat der Phthalsäure ungesetzt wird.
